# EUROPÄISCHE PATENTANMELDUNG

(11) **EP 4 672 256 A1**
(43) Veröffentlichungstag der Anmeldung: **31.12.2025**
(21) Anmeldenummer: 24184320.0
(22) Anmeldetag: 25.06.2024
(51) Int. Cl.: G16H 30/00, G16H 30/20, G16H 30/40, G16H 40/63, G16H 50/20

(54) **COMPUTERIMPLEMENTIERTES VERFAHREN ZUR ERZEUGUNG EINES PROTOKOLLS UND/ODER EINER AUSGABE, DIE AUF EINE ERZEUGUNG ODER MODIFIZIERUNG EINES PROTOKOLLS GERICHTET IST, MEDIZINISCHE EINRICHTUNG, COMPUTERLESBARES SPEICHERMEDIUM UND COMPUTERIMPLEMENTIERTES VERFAHREN ZUR ERZEUGUNG EINES TRAINIERTEN MODELLS**

(71) Anmelder: Siemens Healthineers AG, 91301 Forchheim (DE)
(72) Erfinder: Wohlers, Julian, 91052 Erlangen (DE); Zeller, Mario, 91054 Erlangen (DE); Grodzki, David, 91058 Erlangen (DE); Möck, Thomas, 90443 Nürnberg (DE); Schneider, Rainer, 91315 Höchstadt (DE); Stranjak, Armin, 91080 Uttenreuth (DE); Würfl, Tobias, 91054 Erlangen (DE)
(74) Vertreter: Siemens Healthineers Patent Attorneys

(57) **Zusammenfassung**

Computerimplementiertes Verfahren zur Erzeugung eines Protokolls und/oder einer Ausgabe, die auf eine Erzeugung oder Modifizierung eines Protokolls gerichtet ist, medizinische Einrichtung, computerlesbares Speichermedium und computerimplementiertes Verfahren zur Erzeugung eines trainierten Modells

Die Erfindung betrifft ein computerimplementiertes Verfahren zur Erzeugung eines Protokolls (9) und/oder einer Ausgabe (23), die auf eine Erzeugung oder Modifizierung eines Protokolls (9) gerichtet ist, wobei das Protokoll (9) eine Durchführungsvorschrift zur Steuerung des Ablaufs der Durchführung eines medizinischen Bildgebungsverfahrens mittels einer medizinischen Bildgebungseinrichtung (5) darstellt und wenigstens einen veränderbaren Parameter (18, 19) des Bildgebungsverfahrens zu dessen Durchführung vorgibt, wobei das Verfahren die folgenden Schritte umfasst:
- Vorgeben wenigstens eines Eingangsdatensatzes (10, 12, 14, 15, 16, 22, 25), der wenigstens eine Vorgabe für das Bildgebungsverfahren betrifft,
- Auswerten des wenigstens eines Eingangsdatensatzes (10, 12, 14, 15, 16, 22, 25), wobei in Abhängigkeit des Ergebnisses dieses Auswertens das Protokoll (9) und/oder die Ausgabe (23) erzeugt wird, wobei dieses Auswerten unter Verwendung eines trainierten und mittels eines maschinellen Lernens erzeugten Sprachmodells (8) erfolgt.

## Beschreibung

Computerimplementiertes Verfahren zur Erzeugung eines Protokolls und/oder einer Ausgabe, die auf eine Erzeugung oder Modifizierung eines Protokolls gerichtet ist, medizinische Einrichtung, computerlesbares Speichermedium und computerimplementiertes Verfahren zur Erzeugung eines trainierten Modells

Die vorliegende Erfindung betrifft ein computerimplementiertes Verfahren zur Erzeugung eines Protokolls und/oder einer Ausgabe, die auf eine Erzeugung oder Modifizierung eines Protokolls gerichtet ist, wobei das Protokoll eine Durchführungsvorschrift zur Steuerung des Ablaufs der Durchführung eines medizinischen Bildgebungsverfahrens mittels einer medizinischen Bildgebungseinrichtung darstellt und wenigstens einen veränderbaren Parameter des Bildgebungsverfahrens zu dessen Durchführung vorgibt.

Zur Durchführung respektive Steuerung des Ablaufs des medizinischen Bildgebungsverfahrens ist typischerweise ein Protokoll vorgesehen, anhand dem die im Zuge dieser Durchführung vorzunehmenden Einstellungen und veränderbaren Schritte vorgegeben werden. Das computerlesbare Protokoll bildet einen Datensatz, der auf einem Speichermedium hinterlegt ist und Anweisungen umfasst, die, wenn diese mittels einer Verarbeitungseinrichtung ausgeführt werden, die Generierung und Ausgabe von Steuerbefehlen bewirken, mittels denen die Durchführung des Bildgebungsverfahrens erfolgt. Abgesehen von der Positionierung des Patienten erfolgt die Durchführung des Bildgebungsverfahrens typischerweise komplett mittels des Protokolls, ohne dass das Zutun eines Anwenders erforderlich ist. In der Praxis wird das Protokoll oft auch als Workflow bezeichnet.

Die Erfindung stellt sich die Aufgabe, ein verbessertes Konzept im Zusammenhang mit der Erzeugung respektive Generierung eines Protokolls anzugeben, mittels dem der Ablauf der Durchführung eines medizinischen Bildgebungsverfahrens steuerbar ist.

Erfindungsgemäß gelöst wird die Aufgabe bei einem Verfahren der eingangs genannten Art dadurch, dass dieses die folgenden Schritte umfasst:
- Vorgeben wenigstens eines Eingangsdatensatzes, der wenigstens eine Vorgabe für das Bildgebungsverfahren betrifft,
- Auswerten des wenigstens eines Eingangsdatensatzes, wobei in Abhängigkeit des Ergebnisses dieses Auswertens das Protokoll und/oder die Ausgabe erzeugt wird, wobei dieses Auswerten unter Verwendung eines trainierten und mittels eines maschinellen Lernens erzeugten Sprachmodells erfolgt.

Die Erfindung ermöglicht es, vermittels der Nutzung der auf einer künstlichen Intelligenz basierenden Technologie des Sprachmodells ein Protokoll zu erzeugen, das eine möglichst vorteilhafte Durchführungsvorschrift für das Bildgebungsverfahren realisiert, die spezifisch an die Umstände des jeweils durchzuführenden Bildgebungsverfahrens angepasst ist. So können konkrete Umstände des jeweils durchzuführenden Bildgebungsverfahrens Berücksichtigung finden, betreffend etwa den jeweiligen Patienten und/oder konkrete ärztliche Vorgaben und/oder technische Gegebenheiten der Bildgebungseinrichtung und dergleichen. Diese Umstände werden im Rahmen des wenigstens einen Eingangsdatensatzes bereitgestellt, auf den das Sprachmodell angewendet wird. Bei dem medizinischen Bildgebungsverfahren kann es sich beispielsweise um ein Magnetresonanztomographie-Bildgebungsverfahren oder um ein Computertomografie-Bildgebungsverfahren handeln.

Das Auswerten des wenigstens einen Eingangsdatensatzes kann zur unmittelbaren Erzeugung des Protokolls führen, das somit das Ergebnis dieses Auswertens darstellt. Alternativ kann das Auswerten des wenigstens einen Eingangsdatensatzes zur Erzeugung der Ausgabe führen, von der das Protokoll lediglich mittelbar abhängt. Bezüglich dieser Alternative ist denkbar, dass das Protokoll seitens des Anwenders generiert respektive fertiggestellt wird. Im Rahmen der Ausgabe werden somit bevorzugt Empfehlungen und/oder Vorschläge und/oder Aufforderungen für den Anwender vorgegeben, die auf die Fertigstellung des Protokolls durch den Anwender gerichtet sind. Der Anwender ist zumeist medizinisches Fachpersonal, das bezüglich der Durchführung des Bildgebungsverfahrens respektive der Handhabung und Bedienung der Bildgebungseinrichtung entsprechend fachkundig ist.

Besonders liegt der wenigstens eine Eingangsdatensatz und/oder das Protokoll und/oder die Ausgabe als ein digitalisierter, insbesondere serieller, Text beziehungsweise in einem Textformat vor. So kann der wenigstens eine Eingangsdatensatz eine sprachliche Aufforderung realisieren, die die zur Durchführung des Auswertens erforderlichen Informationen umfasst. Solche dem Sprachmodell zugeführte Aufforderungen werden typischerweise auch als "Prompts" bezeichnet. Denkbar sind auch digitale Grafik- respektive Bildformate, etwa wenn der Eingangsdatensatz anhand eines handschriftlichen Schriftstücks oder Ausdrucks erzeugt wird. So ist denkbar, dass der Eingangsdatensatz im Zuge des Auswertens oder vor dem Auswerten entsprechend umformatiert wird, etwa in ein Text- oder ein Grafik- respektive Bildformat.

Im Rahmen der vorliegenden Erfindung vorgesehene Modelle werden häufig auch als große Sprachmodelle oder Large Language Models, abgekürzt mit "LLM", bezeichnet. Zur Auswertung sprachlicher Eingaben sind Sprachmodelle wie etwa GPT-3 besonders gut geeignet. Ein trainiertes Sprachmodell realisiert eine auf einer künstlichen Intelligenz beruhende, linguistische Datenverarbeitung. Einer der zentralen Kerne solcher Modelle besteht darin, Sprachen wie etwa die menschliche Sprache unter Berücksichtigung grammatikalischer Regeln und semantischer Sinngehalte zu analysieren, zu verstehen und zu imitieren. Sprachmodelle ermöglichen es, gesprochene Sprache, Handschriften, Texte oder Bilder von Texten inhaltlich auszuwerten und somit Interpretationen durchzuführen und Schlussfolgerungen zu ziehen. Auch die Ausgabe respektive das Ergebnis der entsprechenden Analyse liegt typischerweise als eine Sprache respektive in Textform vor.

Gegenüber einer durch den Anwender erfolgenden Erzeugung des Protokolls bewirkt die Verwendung des trainierten Sprachmodells eine Effizienzsteigerung hinsichtlich der hierzu benötigten Ressourcen, insbesondere Zeit. Weiterhin wird vorteilhaft bewirkt, dass mittels der künstlichen Intelligenz eine Berücksichtigung mannigfaltiger und für den menschlichen Anwender unüberschaubarer Umstände und Zusammenhänge ermöglicht wird. Folglich ist das das Ergebnis der Auswertung des Eingangsdatensatzes, also das Protokoll, möglichst erfolgversprechend hinsichtlich des Zweckes der Durchführung des Bildgebungsverfahrens.

Der Parameter oder wenigstens einer der Parameter kann ein Messparameter sein, der eine während der Durchführung des Bildgebungsverfahrens vorliegende physikalische oder geometrische Gegebenheit betrifft. Sofern es sich bei dem Bildgebungsverfahren um ein Magnetresonanztomographie-Bildgebungsverfahren handelt, bei dem wenigstens ein sogenanntes Tomogramm als die Patientenaufnahme erzeugt wird, dann kann der eine physikalische Gegebenheit betreffende Messparameter eine Repetitionszeit oder einer Echozeit oder einer Inversionszeit betreffen, also insbesondere einen Kontrast beziehungsweise eine Wichtung des jeweiligen Tomogramms. Der eine geometrische Gegebenheit betreffende Messparameter kann eine für die Patientenaufnahme oder das Tomogramm vorgesehene Bildgebungsebene, insbesondere eine räumliche Lage oder eine räumliche Orientierung oder eine Schichtdicke oder eine Beschleunigung der Bildgebungsebene oder eine Auflösung oder eine Matrixgröße der resultierenden Patientenaufnahme betreffen.

Der Parameter oder wenigstens einer der Parameter kann ein Schrittparameter sein, der eine zeitliche Dauer und/oder einen Startzeitpunkt und/oder einen Endzeitpunkt wenigstens eines Messschritts betrifft, der im Rahmen der Durchführung des Bildgebungsverfahrens durchgeführt wird. So kann während der Durchführung des Bildgebungsverfahrens die sukzessive Durchführung mehrerer Messschritte vorgesehen sein, für die der Messparameter oder wenigstens einer der Messparameter jeweils separat vorgegeben und neu eingestellt wird, wobei die Messschritte anhand des wenigstens einen Schrittparameters vorgegeben werden.

Der Eingangsdatensatz oder wenigstens einer der Eingangsdatensätze kann spezifisch den jeweiligen Patienten betreffen, also eine patientenspezifische Information sein. Nachfolgend werden einige diesbezügliche Möglichkeiten erläutert, wobei auch denkbare Zusammenhänge genannt werden. Diesbezüglich sei nochmals erwähnt, dass die künstliche Intelligenz neben den nachfolgend erläuterten Zusammenhängen auch weitere, unbekannte und verdeckte Zusammenhänge identifizieren kann.

Denkbar ist, dass der Eingangsdatensatz oder wenigstens einer der Eingangsdatensätze eine im Rahmen einer früheren Durchführung eines medizinischen Bildgebungsverfahrens erfasste Patientenaufnahme betrifft oder betreffen. Durch einen Vergleich der aktuell zu erzeugenden mit der älteren beziehungsweise im Rahmen der früheren Durchführung des Bildgebungsverfahrens erfasste Patientenaufnahme wird eine gegebenenfalls eingetretene Veränderung bei dem Patienten erkennbar. Die Auswertung respektive Festlegung des wenigstens einen Parameters kann derart erfolgen, dass eine Vergleichbarkeit der älteren Patientenaufnahme mit der aktuell zu erzeugenden Patientenaufnahme ermöglicht ist. Der Eingangsdatensatz kann über ein Bilddatenarchivierungs- und Kommunikationssystem der Institution, in der das Bildgebungsverfahren durchgeführt wird, insbesondere des jeweiligen Krankenhauses, abgerufen und bereitgestellt werden. Derartige Bilddatenarchivierungs- und Kommunikationssysteme werden häufig auch mit der Abkürzung "PACS" bezeichnet.

Denkbar ist, dass der Eingangsdatensatz oder wenigstens einer der Eingangsdatensätze einen Auszug aus einer elektronischen Patientenakte betrifft oder betreffen, die typischerweise auch mit der Abkürzung "EMR" bezeichnet wird. Die elektronische Patientenakte respektive Krankenakte umfasst typischerweise alle für den jeweiligen Patienten vorliegenden Daten und Informationen. Diese umfassen bevorzugt personenbezogene Daten wie etwa das Alter und/oder das Geschlecht und/oder das Gewicht und/oder die Größe des Patienten sowie Gesundheitsdaten wie etwa Angaben hinsichtlich früherer Erkrankungen und/oder Behandlungen und/oder Medikationen. Die Auswertung respektive Festlegung des wenigstens einen Parameters kann derart erfolgen, dass ein bereits zuvor von einer Erkrankung betroffener Körperabschnitt des Patienten gezielt im Rahmen des Bildgebungsverfahrens erfasst wird.

Der Eingangsdatensatz oder wenigstens einer der Eingangsdatensätze kann eine auf einen aktuellen Zustand des Patienten gerichtete Patientenabfrage gerichtet sein. So kann die Patientenabfrage vorbereitend für die Durchführung des Bildgebungsverfahrens durchgeführt werden, etwa indem der Patient einen Fragebogen ausfüllt. Hierbei kann der Patient für die aktuelle Diagnostik relevante Fragen beantworten, etwa hinsichtlich eines etwaigen Rauchverhaltens und/oder Details bezüglich etwaiger Beschwerden. Die Auswertung respektive Festlegung des wenigstens einen Parameters kann derart erfolgen, dass ein von dem Auftreten von Symptomen betroffener Körperabschnitt gezielt bei dem Bildgebungsverfahren erfasst wird.

Denkbar ist auch, dass der Eingangsdatensatz oder wenigstens einer der Eingangsdatensätze eine auf die Durchführung des Bildgebungsverfahrens gerichtete, ärztliche Vorgabe betrifft. Die ärztliche Vorgabe kann eine medizinische Diagnose oder Verdachtsdiagnose betreffen. Die Auswertung respektive Festlegung des wenigstens einen Parameters kann derart erfolgen, dass ein von einer diagnostizierten Krankheit betroffener Körperabschnitt des Patienten gezielt im Rahmen des Bildgebungsverfahrens erfasst wird. So kann etwa die ärztliche Vorgabe darin bestehen, dass die Bildgebung hinsichtlich eines spezifischen Organs, etwa der Lunge oder dem Herz, oder einem Körperteil, etwa dem Kopf, durchzuführen ist. Auch ist denkbar, dass die ärztliche Vorgabe unmittelbar den Parameter oder wenigstens einen der Parameter angibt.

Bezüglich des die ärztliche Vorgabe betreffenden Eingangsdatensatzes wird das Problem überwunden, dass ein ansonsten bislang häufiger Zwischenschritt nicht mehr erforderlich ist, bei dem der Anwender die ärztliche Vorgabe interpretieren muss und anhand hiervon die Parameter festlegen respektive das Protokoll erzeugen muss. Dies ist jedoch häufig mit Unsicherheiten verbunden, da für die ärztlichen Vorgaben, die mitunter von institutsfremden Ärzten stammen, keine einheitliche Form vorgesehen ist. Folglich ist bezüglich des Anwenders häufig eine jahrelange Berufserfahrung erforderlich, um Parameter vorzugeben, sodass eine für die vorliegenden Zwecke brauchbare Bildgebung sichergestellt wird.

Die ärztliche Vorgabe kann über ein Radiologie-Informationssystem der jeweiligen Institution, dass kurz auch als "RIS" bezeichnet wird, vorgegeben werden. So kann die ärztliche Vorgabe in digitaler Form, etwa in einem Textformat, vorliegen. Denkbar ist jedoch auch, dass die ärztliche Vorgabe in Papierform vorliegt, also beispielsweise als ein Ausdruck oder handschriftlich. In diesem Fall kann dieses Schriftstück digitalisiert respektive gescannt und mittels dem Sprachmodell zugeführt werden, das diesbezüglich auch hinsichtlich der Erfassung und Auswertung handschriftlicher oder ausgedruckter Texte trainiert wurde.

Erfindungsgemäß ist denkbar, dass wenigstens ein hinterlegtes Standardprotokoll vorgesehen ist, bei dem der Parameter oder wenigstens einer der Parameter standardisiert vorgegeben ist, wobei der Eingangsdatensatz oder wenigstens einer der Eingangsdatensätze das Standardprotokoll oder wenigstens eines der Standardprotokolle ist oder umfasst. So sind bevorzugt mehrere Standardprotokolle auf einer Datenbank einer medizinischen Einrichtung hinterlegt, die die medizinische Bildgebungseinrichtung und gegebenenfalls weitere medizinische Bildgebungseinrichtungen umfasst. Die Standardprotokolle sind bevorzugt jeweils einem Zweck zugeordnet, der im Rahmen der Durchführung des medizinischen Bildgebungsverfahrens mittels des jeweiligen Standardprotokolls erreicht werden soll. So kann das jeweilige Standardprotokoll einer Diagnose beziehungsweise Verdachtsdiagnose oder einem mittels der Bildgebung zu erfassenden Körperabschnitt zugeordnet sein. Grundsätzlich ist das Standardprotokoll unmittelbar und unverändert für die Durchführung des medizinischen Bildgebungsverfahrens verwendbar. Bei dem erfindungsgemäßen Verfahren ist jedoch bevorzugt vorgesehen, dass das Standardprotokoll mittels des Sprachmodells situationsspezifisch anhand der zudem vorliegenden Eingangsdatensätze modifiziert wird.

Denkbar ist, dass der Eingangsdatensatz oder wenigstens einer der Eingangsdatensätze ein eine konkrete anwenderseitige Vorgabe für den Parameter oder für wenigstens einen der Parameter betrifft. Der entsprechende Eingangsdatensatz kann, wie bereits zuvor erläutert wurde, die ärztliche Vorgabe betreffen. Denkbar ist auch, dass der Eingangsdatensatz eine auf den Parameter gerichtete Vorgabe des vor Ort an der medizinischen Bildgebungseinrichtung anwesenden Bedienungspersonals ist, das auf einer konkreten Erfahrung mit der Bildgebungseinrichtung basierende Vorgaben diesbezüglich macht. Konkret kann vorgesehen sein, dass als der Eingangsdatensatz oder als wenigstens einer der Eingangsdatensätze das Standardprotokoll oder eines der Standardprotokolle genutzt wird, das hinsichtlich des Parameters oder hinsichtlich wenigstens einem der Parameter seitens eines Anwenders geändert wurde. So kann vorgesehen sein, dass der Anwender den Parameter über eine Mensch-Maschine-Schnittstelle ändert, die insbesondere eine Ein- und/oder Ausgabeeinrichtung wie ein Touchscreen ist oder umfasst. Diesbezüglich können veränderbare Parameter über eine Eingabemaske durch den Anwender vorgegeben werden.

Insbesondere wenn die konkrete anwenderseitige Vorgabe für den Parameter vorgesehen ist, ist denkbar, dass das Auswerten des wenigstens einen Eingangsdatensatzes auf das Erkennen des Vorliegens einer Konsistenz des wenigstens einen, insbesondere anwenderseitig geänderten, Parameters mit wenigstens einer zur Durchführung des Bildgebungsverfahrens relevanten Größe gerichtet ist. Bezüglich der Durchführung dieser Konsistenzprüfung ist erfindungsgemäß auch denkbar, dass diese für die genannten Standardprotokolle durchgeführt wird. Sofern die Konsistenz des Parameters gegeben ist, dann liegt ein Zustand vor, bei dem der Wert dieses Parameters im Einklang respektive nicht im Widerspruch zu im Zusammenhang mit der Durchführung des Bildgebungverfahrens gegebenen Umständen steht. So kann eine etwaige fehlende Konsistenz für einen Parameter, insbesondere wenn dieser anwenderseitig vorgegeben beziehungsweise verändert wird, übersehen werden, wobei gemäß dieser Ausführungsform das Auswerten des wenigstens einen Eingangsdatensatzes gezielt auf die Erkennung und gegebenenfalls Beseitigung etwaiger Inkonsistenzen gerichtet ist.

Im Rahmen dieser Ausführungsform ist vorgesehen, dass das Auswerten des wenigstens einen Eingangsdatensatzes auf das Erkennen des Vorliegens einer Konsistenz des wenigstens einen Parameters mit wenigstens einer zur Durchführung des Bildgebungsverfahrens relevanten Größe gerichtet ist. So ist denkbar, dass diese Größe denjenigen Eingangsdatensatz betrifft, der spezifisch den jeweiligen Patienten betrifft. So kann anhand des Geschlechts, des Gewichts und der Größe des Patienten überprüft werden, ob eine Überschreitung einer spezifischen Absorptionsrate bei dem Patienten bei der Durchführung des Bildgebungsverfahrens zu erwarten ist, wobei die Konsistenz in diesem Fall nicht vorliegt. Dies kann etwa dann eintreten, wenn eine anwenderseitige Änderung des Parameters dahingehend erfolgt ist, bei der die Repetitionszeit verkürzt und/oder ein Kippwinkel erhöht wird. Weiterhin können, etwa unter Verwendung eines Stimulationsmodells, Vorhersagen über Nervenstimulationen getroffen werden, etwa im Fall einer Änderung der Orientierung der Bildgebungsebene respektive Aufnahmeschicht, wobei das Vorliegen der Konsistenz hiervon abhängen kann. Denkbar ist, dass während der Durchführung des Bildgebungsverfahrens Pausen bezüglich der Atmung des Patienten, also ein Luftanhalten erforderlich sind, wobei die zeitliche Länge dieser Pausen gegebenenfalls von dem wenigstens einen Parameter abhängt. Insbesondere unter zusätzlicher Berücksichtigung der patientenspezifischen Information kann eine Inkonsistenz dann vorliegen, wenn die aus den Parametern resultierende, zeitliche Dauer für die Atempause eine vorgegebene, zulässige Höchstdauer überschreitet. Weiterhin kann überprüft werden, ob die Verwendung eines sogenannten Shims, mittels dem insbesondere eine Homogenisierung des erzeugten Magnetfeldes bewirkt wird, vorliegend eine diesbezügliche Zweckmäßigkeit betreffenden Voraussetzungen entspricht. Diese Überprüfung kann darauf gerichtet sein, ob diese Verwendung bei dem mittels des Bildgebungsverfahrens zu erfassenden Körperabschnitt und/oder einer vorgegebenen Schichtabdeckung zweckmäßig ist.

Denkbar ist, dass das Auswerten des wenigstens eines Eingangsdatensatzes auf das Erkennen des Vorliegens einer Konsistenz des wenigstens einen anwenderseitig geänderten Parameters mit einer Bezeichnung des zur Generierung dieses Eingangsdatensatzes geänderten Standardprotokolls gerichtet ist. In diesen Kontext können Probleme vermieden werden, die auftreten können, wenn die Bezeichnung des Standardprotokolls auch für das erzeugte Protokoll verwendet wird. So kann die vorgenommene Änderung dazu führen, dass diese Bezeichnung nicht mehr konsistent mit den Parametern ist. So kann etwa die Bezeichnung den Text "purely T2-weighted" umfassen, wobei die Repetitionszeit und/oder die Echozeit derart verändert worden sein könnte, dass bei der Durchführung des Bildgebungsverfahrens gemäß dem resultierenden Protokoll keine ausschließliche T2-Wichtung mehr vorliegt. In diesem Fall liegt eine Inkonsistenz zwischen den jeweiligen Parametern und der Bezeichnung des zugehörigen Protokolls vor.

Insbesondere ist denkbar, dass das Auswerten des wenigstens einen Eingangsdatensatzes auf das Erkennen des Vorliegens einer Konsistenz mehrerer der Parameter untereinander gerichtet ist. So kann etwa überprüft werden, ob ausgewählte Spulenkanäle der Schichtabdeckung den Beschleunigungseinstellungen, insbesondere Beschleunigungsfaktoren wie dem PATbeziehungsweise dem SMS-Faktor, entsprechen. Konkret ist denkbar, dass die Konsistenz mehrerer Parameter dann gegeben ist, wenn, insbesondere paarweise, Kombinationen von Parametern erlaubten, insbesondere vorgegebenen, Kombinationen entsprechen.

Sofern das Auswerten des wenigstens einen Eingangsdatensatzes ein Fehlen der Konsistenz ergibt, dann kann die Ausgabe derart erzeugt werden, dass ein Anwender anhand der Ausgabe über das Fehlen der Konsistenz informiert wird. Dem Anwender kann diese Information über die Mensch-Maschine-Schnittstelle ausgegeben werden. Der Anwender kann sodann entscheiden, ob er den betroffenen Parameter dennoch beibehalten oder entsprechend ändern möchte. Besonders bevorzugt ist vorgesehen, dass die Ausgabe einen an den Anwender gerichteten Vorschlag bezüglich einer vorzunehmenden Änderung des jeweiligen Parameters zur Beseitigung der Inkonsistenz umfasst. Die Ausgabe kann in einer tokenisierten Form vorliegen, bei der der oder die Parameter, die bezüglich einer Änderung vorgeschlagen werden, im Rahmen der entsprechenden Token-Kombination konkret angegeben wird. Diese Vorschläge können seitens des Anwenders angenommen und zur automatischen Anpassung des jeweiligen Protokolls weitergeleitet werden.

Erfindungsgemäß ist denkbar, dass die medizinische Bildgebungseinrichtung, und gegebenenfalls wenigstens eine weitere medizinische Bildgebungseinrichtung, zu einer medizinischen Einrichtung gehören. Das bedeutet, dass die medizinische Einrichtung zumindest die medizinische Bildgebungseinrichtung, bevorzugt zudem wenigstens eine weitere medizinische Bildgebungseinrichtung, die der medizinischen Bildgebungseinrichtung entsprechen kann, umfasst. Die medizinische Einrichtung kann ein System, etwa einer jeweiligen Einrichtung respektive eines jeweiligen Krankenhauses, sein, bei dem die Komponenten, insbesondere mehrere medizinische Bildgebungseinrichtungen, miteinander vernetzt sind. Denkbar ist diesbezüglich eine zentrale oder dezentrale Steuerung der Bildgebungseinrichtungen. Bevorzugt umfasst medizinische Einrichtung Datenspeicher- und Kommunikationsmittel, mittels denen insbesondere die bereits oben erläuterten Aspekte bezüglich des Bilddatenarchivierungs- und Kommunikationssysteme (PACS) und des Radiologie-Informationssystems (RIS) realisiert werden.

Bevorzugt ist vorgesehen, dass im Zuge der Durchführung eines medizinischen Bildgebungsverfahrens bei der medizinischen Einrichtung oder der medizinischen Bildgebungseinrichtung jeweils ein Historienprotokoll generiert und gespeichert wird, wobei das Historienprotokoll das für die Durchführung dieses medizinischen Bildgebungsverfahrens benutzte Protokoll oder ein Datensatz betreffend die für die Durchführung dieses medizinischen Bildgebungsverfahrens verwendeten Parameter ist. Gemäß dieser Ausführungsform steht ein Archiv zur Verfügung, das die Historienprotokolle und mithin Informationen bezüglich zeitlich früher stattgefundener Bildgebungsverfahren umfasst. Die hierbei verwendeten Protokolle können unverändert als Historienprotokolle hinterlegt werden. Alternativ können nur ausgewählte Informationen respektive Parameter für die Durchführung des jeweiligen Bildgebungsverfahrens als der Datensatz abgespeichert werden. Zur Generierung des jeweiligen Datensatzes können die zu speichernden Informationen aus dem Dateinamen des jeweiligen Protokolls ermittelt werden. Zusätzlich oder alternativ kann zu diesem Zweck ein Header des benutzten Protokolls ausgelesen und hieraus die Informationen ermittelt werden. So kann das Protokoll als eine Datei im sogenannten DICOM-Format vorliegen, wobei die Abkürzung DICOM für "Digital Imaging and Communications in Medicine" steht, wobei Dateien dieses Formats typischerweise entsprechende Header-Dateien aufweisen.

Bevorzugt ist der Eingangsdatensatz oder wenigstens einer der Eingangsdatensätze die derart generierten Historienprotokolle ist oder umfasst. Mithin ist im Rahmen dieser Ausführungsform bevorzugt vorgesehen, dass das Auswerten des wenigstens einen Eingangsdatensatzes auf das Erkennen einer zeitlichen Entwicklung des Parameters oder wenigstens einem der Parameter gerichtet ist. So werden bei einer medizinischen Institution nicht selten Änderungen bezüglich der Durchführungen von Bildgebungsverfahren vorgenommen, insbesondere aufgrund neuer Erfahrungen, Vorgaben oder Empfehlungen. Diese Änderungen werden nicht immer lückenlos und nachvollziehbar erfasst, sodass auch Standardprotokolle diesbezüglich nicht unbedingt stets aktuell gehalten werden. Da mit der Verwendung der Historienprotokolle als die Eingangsdatensätze eine Datenbasis zur Verfügung steht, anhand der etwaige Trends respektive Änderungen identifiziert beziehungsweise erkannt werden können, kann die aktuelle Erzeugung des Protokolls oder die Ausgabe unter Berücksichtigung dieser Umstände erfolgen.

Wie bereits oben erläutert wurde, kann das Auswerten des Eingangsdatensatzes respektive der Eingangsdatensätze auf das Erkennen des Vorliegens einer Konsistenz gerichtet sein. Diesbezüglich ist denkbar, dass das Auswerten des wenigstens eines Eingangsdatensatzes auf das Erkennen des Vorliegens einer Konsistenz des wenigstens einen geänderten Parameters mit dessen zeitlicher Entwicklung gerichtet ist. Anders ausgedrückt wird im Rahmen dieser Ausführungsform die Konsistenz dahingehend überprüft, ob ein Wert für den, insbesondere anwenderseitig geänderten, Parameter mit einem zeitlichen Verhalten dieses Parameters bezüglich vorher durchgeführter Bildgebungsverfahren in Einklang steht. Anhand der Historienprotokolle kann eine zeitliche Reihe umfassend die Werte dieses Parameters ermittelt werden, wobei überprüft wird, ob der, insbesondere geänderte, Parameter konsistent mit dieser Reihe ist. So kann etwa überprüft werden, ob der Parameter konsistent mit einem anhand der zeitlichen Reihe extrapolierten Wert ist. Denkbar ist auch, dass anhand der zeitlichen Reihe ersichtlich wird, dass sich der Wert für den Parameter zu einem bestimmten Zeitpunkt, insbesondere sprunghaft, geändert hat und im Übrigen jeweils im Wesentlichen konstant blieb, wobei überprüft wird, ob der geänderte Parameter konsistent mit dem nach dem Eintreten dieser Änderung vorliegenden Wert ist respektive diesem entspricht.

Sofern als das Ergebnis der Auswertung das Protokoll erzeugt wird, dann ist denkbar, dass das Protokoll anhand des Ergebnisses der Auswertung automatisch derart erzeugt wird, dass der von der zeitlichen Änderung betroffene Parameter in Einklang mit dieser zeitlichen Änderung gebracht wird. Sofern als das Ergebnis der Auswertung die Ausgabe erzeugt wird, dann ist denkbar, dass der Anwender anhand der Ausgabe über die zeitliche Änderung informiert wird. Gemäß einer Weiterbildung hiervon ist denkbar, dass die Ausgabe eine an den Anwender gerichtete Empfehlung bezüglich einer vorzunehmenden Änderung des wenigstens einen von der zeitlichen Änderung betroffenen Parameters umfasst, sodass der Wert für den von der zeitlichen Änderung betroffene Parameter im Einklang mit dieser zeitlichen Änderung steht.

Die vorliegende Erfindung betrifft ferner eine medizinische Einrichtung zur Durchführung des Verfahrens gemäß obiger Beschreibung. Erfindungsgemäß gelöst wird die Aufgabe bei einer solchen medizinischen Einrichtung dadurch, dass diese eine Bereitstellungseinrichtung, mittels der der wenigstens eine Eingangsdatensatz einer Verarbeitungseinrichtung vorgebbar ist, und die Verarbeitungseinrichtung umfasst, die dazu eingerichtet ist, das trainierte und mittels eines maschinellen Lernens erzeugte Sprachmodell zum Auswerten des wenigstens eines Eingangsdatensatzes auf den wenigstens einen Eingangsdatensatz anzuwenden, wobei hierdurch das Protokoll und/oder die Ausgabe erzeugt wird. Die Bereitstellungseinrichtung kann eine Mensch-Maschine-Schnittstelle umfassen. Alle im Zusammenhang mit dem erfindungsgemäßen Verfahren beschriebenen Vorteile, Merkmale und Aspekte sind gleichermaßen auf die erfindungsgemäße medizinische Einrichtung übertragbar und umgekehrt.

Weiterhin betrifft die vorliegende Erfindung ein computerlesbares Speichermedium. Erfindungsgemäß gelöst wird die Aufgabe der vorliegenden Erfindung bei einem solchen Speichermedium einerseits dadurch, dass diese Anweisungen umfasst, die, wenn diese mittels einer als ein Computer ausgebildeten Verarbeitungseinrichtung ausgeführt werden, die Verarbeitungseinrichtung dazu veranlassen, das Verfahren gemäß obiger Beschreibung durchzuführen. Erfindungsgemäß gelöst wird die Aufgabe der Erfindung bei einem solchen Speichermedium andererseits dadurch, dass diese Anweisungen umfasst, die, wenn diese mittels einer als ein Computer ausgebildeten Verarbeitungseinrichtung ausgeführt werden, die Verarbeitungseinrichtung dazu veranlassen, wenigstens einen vorgegebenen und eine Vorgabe für ein medizinisches Bildgebungsverfahren betreffenden Eingangsdatensatz auszuwerten, wobei in Abhängigkeit des Ergebnisses dieses Auswertens ein Protokoll und/oder eine Ausgabe, die auf eine Erzeugung oder Modifizierung eines Protokolls gerichtet ist, erzeugt wird, wobei dieses Auswerten unter Verwendung eines trainierten und mittels eines maschinellen Lernens erzeugten Sprachmodells erfolgt, wobei das Protokoll eine Durchführungsvorschrift zur Steuerung des Ablaufs der Durchführung eines medizinischen Bildgebungsverfahrens mittels einer medizinischen Bildgebungseinrichtung darstellt und wenigstens einen veränderbaren Parameter des Bildgebungsverfahrens zu dessen Durchführung vorgibt. Alle im Zusammenhang mit dem erfindungsgemäßen Verfahren und der erfindungsgemäßen medizinischen Einrichtung erläuterten Vorteile, Merkmale und Aspekte sind gleichermaßen auf das erfindungsgemäße computerlesbare Speichermedium übertragbar und umgekehrt.

Schließlich betrifft die vorliegende Erfindung ein computerimplementiertes Verfahren zur Erzeugung eines trainierten Modells, das im Rahmen der Durchführung des Verfahrens gemäß obiger Beschreibung als das trainierte Sprachmodell verwendbar ist, wobei das Verfahren die folgenden Schritte umfasst:
- Vorgeben wenigstens eines Trainings-Eingangsdatensatzes,
- Vorgeben eines Trainings-Ergebnisses, das dem wenigstens einen Trainings-Eingangsdatensatz zugeordnet wird,
- Trainieren eines Modells basierend auf dem wenigstens einen Trainings-Eingangsdatensatz und dem Trainings-Ergebnis, wodurch das trainierte Modell erhalten wird.

Hinsichtlich des Sprachmodells erfolgt mithin ein Training respektive maschinelles Lernen. So realisiert das trainierte Modell kognitive Funktionen, die dem Vorbild menschlichen Denkens entsprechen oder zumindest ähneln. Das Modell ist mittels der Durchführung des Trainings grundsätzlich in der Lage, bislang unerkannte Zusammenhänge und Muster aufzudecken und zu nutzen. So kann die Bestimmung von Größen und/oder Umständen und/oder Zusammenhängen mittels des Modells über die Durchführung des Trainings weiterentwickelt und verbessert werden. Bei dem Training werden bevorzugt separate Trainingsschritte respektive -zyklen nacheinander durchgeführt, wobei sich das Modell stets verbessert. Konkret kann ein überwachtes Training durchgeführt werden, wobei grundsätzlich auch die Durchführung eines nicht überwachten Trainings denkbar ist.

Als die Trainings-Eingangsdatensätze können reale, in der Vergangenheit vorliegende Datensätze respektive Eingangsdatensätze verwendet werden. Falls das überwachte Training durchgeführt wird, dann können als brauchbar befundene, hieraus nutzerseitig vorgegebene Protokolle oder Anweisungen als Ideallösungen darstellende Trainings-Ergebnisse vorgegeben werden. Die im Rahmen des Trainings mittels des Modells generierten Ergebnisse können hiermit verglichen werden, wobei als Zielvorgabe eine Minimierung der Abweichungen zwischen den Trainings-Ergebnissen und den generierten Ergebnissen vorgegeben wird. Bei der Verwendung eines auf ein neutrales Netzwerk basierenden Modells werden die Eingangsdatensätze respektive die Trainings-Eingangsdatensätze über eine Eingangslage des Modells selbigen zugeführt, wobei die Ergebnisse der Auswertung über eine Ausgangslage des Modells bereitgestellt werden. Dazwischen kann eine große Anzahl weiterer Lagen vorhanden sein, umfassend jeweils eine gewisse Anzahl an Knoten, zwischen denen Verbindungen zur Realisierung eines neuralen Netzes im Rahmen des Trainings ausgebildet werden. Details diesbezüglich sind der Fachperson hinlänglich bekannt und werden daher, da diese den Kern der vorliegenden Erfindung nicht weiter tangieren, nicht näher dargelegt. Im Übrigen sei angemerkt, dass alle im Zusammenhang mit dem eingangs erläuterten erfindungsgemäßen Verfahren, der erfindungsgemäßen medizinischen Einrichtung und dem erfindungsgemäßen computerlesbaren Speichermedium erläuterten Vorteile, Merkmale und Aspekte gleichermaßen auf dieses erfindungsgemäße Verfahren übertragbar sind und umgekehrt.

Weitere Vorteile, Merkmale und Einzelheiten der Erfindung ergeben sich aus den nachfolgend dargelegten Ausführungsbeispielen sowie anhand der Figuren. Diese zeigen schematisch:
Fig. 1: eine schematische Blockdarstellung einer erfindungsgemäßen medizinischen Einrichtung gemäß einem Ausführungsbeispiel, umfassend ein erfindungsgemäßes computerlesbare Speichermedium gemäß einem Ausführungsbeispiel, und
Fig. 2: ein Flussdiagramm eines erfindungsgemäßen Verfahrens gemäß mehrerer Ausführungsbeispiele, das bei der in der Fig. 1 gezeigten erfindungsgemäßen medizinischen Einrichtung durchgeführt wird.

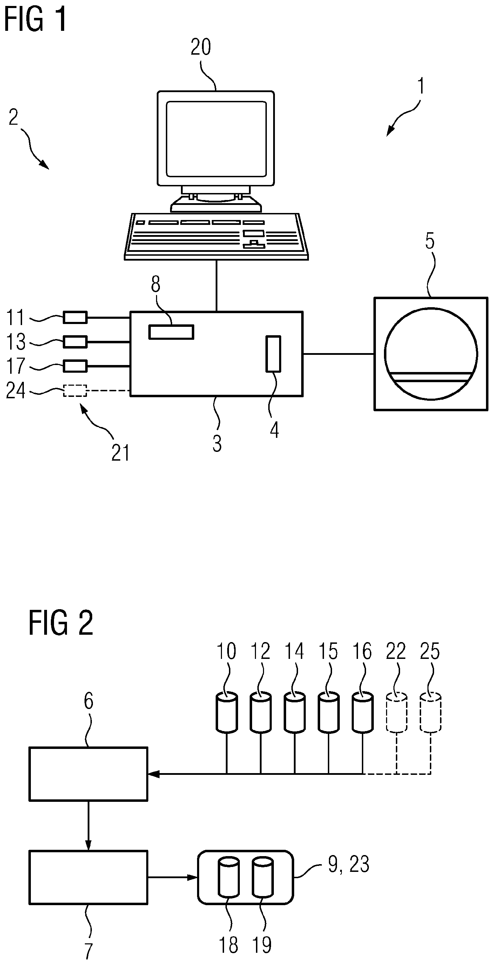

Fig. 1 zeigt eine schematische Darstellung einer erfindungsgemäßen medizinischen Einrichtung 1 gemäß einem Ausführungsbeispiel. Gemäß diesem Blockdiagramm umfasst die medizinische Einrichtung 1 eine Bereitstellungseinrichtung 2, die eine als ein Touchscreen ausgebildete Mensch-Maschine-Schnittstelle 20 aufweist. Ferner umfasst die Einrichtung 1 eine als ein Computer vorgesehene Verarbeitungseinrichtung 3, die ein erfindungsgemäßes computerlesbares Speichermedium 4 gemäß einem Ausführungsbeispiel umfasst. Die Verarbeitungseinrichtung 3 ist dazu eingerichtet, eine medizinische Bildgebungseinrichtung 5 der medizinischen Einrichtung 1 im Rahmen der Durchführung eines medizinischen Bildgebungsverfahrens anzusteuern. Obgleich die medizinische Einrichtung 1 neben der medizinischen Bildgebungseinrichtung 5 weitere medizinische Bildgebungseinrichtungen aufweist, die der medizinischen Bildgebungseinrichtung 5 entsprechen, sind diese aus Gründen der Übersichtlichkeit in der Fig. 1 nicht näher dargestellt. Beispielhaft ist der Bildgebungseinrichtungen 5 jeweils eine Bereitstellungseinrichtung 2 zugeordnet, wobei die Verarbeitungseinrichtung 3 ein Zentralcomputer für alle Bildgebungseinrichtungen 5 ist.

Die medizinische Bildgebungseinrichtung 5 ist, ohne Beschränkung der Allgemeinheit, eine Magnetresonanztomografie-Bildgebungseinrichtung, kann jedoch auch eine Computertomografie-Bildgebungseinrichtung oder dergleichen sein. Mittels der medizinischen Bildgebungseinrichtung 5 ist ein medizinisches Bildgebungsverfahren durchführbar, in dessen Rahmen als Patientenaufnahmen Tomogramme erstellbar sind, wobei das erfindungsgemäße Verfahren auf die Durchführung einer solchen Bildgebungsverfahrens gerichtet ist.

Nachfolgend wird anhand der in Fig. 1 dargestellten medizinischen Einrichtung 1 sowie anhand des in Fig. 2 gezeigten Flussdiagramms ein erfindungsgemäßes Verfahren gemäß einem ersten Ausführungsbeispiel erläutert. Dieses computerimplementierte Verfahren wird seitens der Verarbeitungseinrichtung 3 ausgeführt. So umfasst das Speichermedium 4 Anweisungen, die mittels der Verarbeitungseinrichtung 3 ausgeführt werden und diese dazu veranlassen, Schritte des nachfolgend erläuterten Verfahrens durchzuführen.

In einem ersten Schritt 6 des Verfahrens werden mittels der Bereitstellungseinrichtung 2 Eingangsdatensätze 10, 12, 14, 15, 16 vorgegeben, die Vorgaben für die Durchführung des Bildgebungsverfahrens betreffen. In einem zweiten Schritt 7 werden die Eingangsdatensätze 10, 12, 14, 15, 16 unter Verwendung eines trainierten und mittels eines maschinellen Lernens erzeugten Sprachmodells 8, das auf der Verarbeitungseinrichtung 3 implementiert ist, ausgewertet, In Abhängigkeit der Ergebnisse dieser Auswertung wird ein Protokoll 9 erzeugt, dass eine Durchführungsvorschrift oder, mit anderen Worten, einen Workflow bezüglich der Durchführung des Bildgebungsverfahrens realisiert. Das Protokoll 9 umfasst mehrere veränderbare Parameter 18, 19, in Abhängigkeit derer das Bildgebungsverfahren durchgeführt wird. Nachfolgend werden Details bezüglich der Schritte 6, 7, insbesondere hinsichtlich der Eingangsdatensätze 10, 12, 14, 15, 16 sowie des Protokolls 9, erläutert.

So wird in Schritt 6 ein Eingangsdatensatz 10 vorgegeben, der auf die Durchführung des Bildgebungsverfahrens gerichtete, ärztliche Vorgaben betrifft. Der Eingangsdatensatz 10 kann eine medizinische Diagnose oder Verdachtsdiagnose und/oder einen von einer gegebenenfalls diagnostizierten Krankheit betroffenen Körperabschnitt eines Patienten betreffen. Zudem oder alternativ kann die ärztliche Vorgabe unmittelbar wenigstens einen der Parameter 18, 19 vorgeben. Die ärztliche Vorgabe kann digitalisiert, etwa in einem Textformat, als der Eingangsdatensatz 10 vorgegeben werden, etwa über ein Radiologie-Informationssystem 11 ("RIS") der jeweiligen Institution respektive des jeweiligen Krankenhauses. Das Radiologie-Informationssystem 11 kann mithin als ein Teil der Bereitstellungseinrichtung 2 betrachtet werden. Denkbar ist auch, dass die ärztliche Vorgabe zunächst in Papierform vorliegt, insbesondere handschriftlich oder als ein Ausdruck. In diesem Fall wird dieses Schriftstück digitalisiert, etwa eingescannt, und mittels des Sprachmodells, das auch hinsichtlich der Texterfassung handschriftlicher oder ausgedruckter Texte trainiert wurde, ausgewertet.

Weiterhin werden als Eingangsdatensätze 12 Patientenaufnahmen vorgegeben, die im Rahmen früheren Durchführungen medizinischer Bildgebungen erfasst wurden. Konkret werden diesbezüglich etwa ältere Röntgenaufnahme beziehungsweise Tomogramme abgerufen. Die Eingangsdatensätze 12 werden über ein Bilddatenarchivierungs- und Kommunikationssystem 13 ("PACS") der jeweiligen Institution abgerufen und bereitgestellt, das mithin ebenfalls als ein Teil der Bereitstellungseinrichtung 2 betrachtet werden kann.

Überdies wird als ein Eingangsdatensatz 14 eine elektronische Patientenakte respektive elektronische Krankenakte ("EMR") abgerufen, die alle für den Patienten vorliegenden Daten und Informationen umfasst. So kann der die elektronische Patientenakte betreffende Eingangsdatensatz 14 mitunter auch die die Patientenaufnahmen betreffenden Eingangsdatenätze 12 umfassen. Weiterhin umfasst der Eingangsdatensatz 14 personenbezogene Daten, nämlich das Alter, das Geschlecht, das Gewicht und die Größe sowie Gesundheitsdaten des Patienten, etwa Angaben hinsichtlich früherer Erkrankungen, Behandlungen, Medikationen und dergleichen.

Ein vorgegebener Eingangsdatensatz 15 betrifft eine auf einen aktuellen Zustand des Patienten gerichtete Patientenabfrage, die in Vorbereitung zur Durchführung des Bildgebungsverfahrens mittels des Ausfüllens eines Fragebogens erhoben wurde. Hierbei hat der Patient bezüglich der aktuellen Diagnostik sowie der Durchführung des Bildgebungsverfahrens relevante Fragen beantwortet. Beispielhaft liegt auch dieser Fragebogen zunächst in Papierform vor, der zur Generierung des Eingangsdatensatzes 15 digitalisiert wird.

Ein vorgegebener Eingangsdatensatz 16 betrifft ein hinterlegtes Standardprotokoll, bei dem die abänderbaren Parameter 18, 19 standardisiert vorgegeben sind. Hierzu wird auf eine Datenbank 17 der medizinischen Einrichtung 1 zurückgegriffen, auf der mehrere solcher Standardprotokolle abgespeichert und hinterlegt sind. Die Datenbank 17 realisiert einen Bildgebungs-Protokollmanager, der mithin ebenfalls als ein Teil der Bereitstellungseinrichtung 2 betrachtet werden kann. Die auf der Datenbank 17 hinterlegten Standardprotokolle sind jeweils eine Diagnose und einem Körperabschnitt zugeordnet, der bei der Durchführung des mittels des jeweiligen Standardprotokolls durchgeführten Bildgebungsverfahren erfasst wird.

Anhand der Eingangsdatensätze 10, 12, 14, 15, 16 werden im Schritt 7 mittels des Sprachmodells die Parameter 18, 19 bestimmt und das Protokoll 9 generiert. Ausgangspunkt hierfür sind die Werte für die Parameter 18, 19, wie diese anhand des das jeweilige Standardprotokoll betreffenden Eingangsdatensatzes 16 vorgesehen sind. Diese werden in Abhängigkeit der übrigen Eingangsdatensätze 10, 12, 14, 15 gegebenenfalls fallspezifisch angepasst und verändert. Als die Parameter 18, 19 sind Messparameter 18 und Schrittparameter 19 vorgesehen.

Die Messparameter 18 betreffen die im Rahmen der Durchführung eines Magnetresonanztomografie-Bildgebungsverfahrens typischerweise änder- und einstellbaren Größen. Hinsichtlich der physikalischen Größen ist diesbezüglich eine Repetitionszeit, eine Echozeit und eine Inversionszeit denkbar. Hinsichtlich der geometrischen Größen sind diesbezüglich Aspekte denkbar, die eine für die Patientenaufnahme respektive das Tomogramm vorgesehene Bildgebungsebene betreffen. So ist diesbezüglich eine räumliche Lage oder Orientierung und eine Schichtdicke und eine Beschleunigung der Bildgebungsebene und eine Auflösung und eine Matrixgröße der resultierenden Patientenaufnahme denkbar.

Die Schrittparameter 19 betreffen jeweils eine zeitliche Dauer sowie einen Start- oder einen Endzeitpunkt des mittels des jeweiligen Schrittparameters 19 spezifizierten Messschritts. So werden die Messschritte während der Durchführung des Bildgebungsverfahrens sukzessive durchgeführt, wobei die Messparameter 18 für jeden der Messschritte jeweils separat vorgegeben und gegebenenfalls geändert werden. Zusammenfassend ist für jeden der Messschritte anhand der Schrittparameter 19 das diesem Messschritt jeweils zugehörige Zeitintervall und anhand der Messparameter 18 die diesem Messschritt jeweils zugehörigen physikalischen und geometrischen Gegebenheiten vorgegeben.

Nachfolgend wird das erfindungsgemäße Verfahren gemäß einem zweiten Ausführungsbeispiel erläutert. Hierzu wird auf die in Fig. 1 gezeigte Einrichtung 1 Bezug genommen, wobei auch die anhand der Fig. 2 erläuterten Verfahrensschritte bei dem zweiten Ausführungsbeispiel vorgesehen sein können. Die nachfolgend erläuterten Aspekte sind zusätzlich oder alternativ bei dem erfindungsgemäßen Verfahren gemäß dem ersten Ausführungsbeispiel denkbar.

So ist bezüglich des Verfahrens gemäß dem zweiten Ausführungsbeispiel vorgesehen, dass der Eingangsdatensatz 22, der in Fig. 2 gestrichelt angedeutet ist, eine konkrete Vorgabe für die Parameter 18, 19 betrifft, wobei die Vorgabe seitens eines Anwenders vorgegeben wurde. Der Anwender ist insbesondere das medizinische Fachpersonal, das die Bildgebungseinrichtung 5 bedient und bei der Durchführung des Bildgebungsverfahrens vor Ort ist. Konkret wird der Eingangsdatensatz 22 aus dem das Standardprotokoll betreffenden Eingangsdatensatz 16 generiert, indem die anhand des Standardprotokolls standardisiert vorgegebenen Parameter 18, 19 durch den Anwender und über die Mensch-Maschine-Schnittstelle 20 modifiziert respektive geändert werden. Hierzu wird dem Anwender über die Mensch-Maschine-Schnittstelle 20 eine Eingabemaske angezeigt, über die die Parameter 18, 19 änderbar sind. Anhand der über die Eingabemaske vorgenommenen Vorgaben für die Parameter 18, 19 wird der Eingangsdatensatz 22 als ein digitalisierter Text erzeugt. So werden die im Zuge dieser Vorgaben vorgenommenen Eingaben in einen seriellen und den Eingangsdatensatz 22 darstellenden Text umgewandelt. Konkret ist dieser Eingangsdatensatz 22 eine sprachliche Aufforderung, also ein sogenannter "Prompt", der dem Sprachmodell 8 zugeführt wird und die zur Durchführung des Auswertens erforderlichen Informationen umfasst.

Die Auswertung der Eingangsdatensätze 10, 12, 14, 15, 16, 22 mittels des Sprachmodells 8 erfolgt nun dahingehend, ob, insbesondere bezüglich der anwenderseitig geänderten Parameter, eine Konsistenz vorliegt. Anders ausgedrückt wird überprüft, ob die Parameter 18, 19 im Einklang untereinander und mit zur Durchführung des Bildgebungsverfahrens relevanter Größen und Umstände stehen. Nachfolgend werden einige konkrete Aspekte hinsichtlich der Konsistenzprüfung erläutert, wobei diese Ausführungen nicht abschließend sind, insbesondere da aufgrund der Verwendung der künstlichen Intelligenz weitere, nicht offensichtliche und seitens der künstlichen Intelligenz erkannte Zusammenhänge Berücksichtigung finden können.

So ist etwa vorgesehen, dass diesbezüglich die Überprüfung der Konsistenz der Parameter 18, 19 mit einer Bezeichnung des zur Generierung des Eingangsdatensatzes 22 anwenderseitig geänderten Standardprotokolls gerichtet ist. So umfasst die Bezeichnung des Standardprotokolls typischerweise beschreibende Elemente, wobei überprüft wird, ob diese in Übereinstimmung mit den anwenderseitig vorgegebenen und/oder mit den mittels dem Sprachmodell 8 generierten Parametern 18, 19 steht, wobei dann Konsistenz gegeben ist.

Weiterhin erfolgt das Auswerten der Eingangsdatensätze 10, 12, 14, 15, 16, 22 dahingehend, dass das Vorliegen der Konsistenz der anwenderseitig geänderten Parameter 18, 19 mit zur Durchführung des Bildgebungsverfahrens relevanten Größen überprüft wird. Diese Größen können etwa das Geschlecht, das Gewicht und die Größe des Patienten betreffen und anhand des die elektronische Krankenakte betreffenden Eingangsdatensatzes 14 vorgegeben sein. Hierbei wird etwa überprüft, ob eine Überschreitung einer spezifischen Absorptionsrate bei dem Patienten bei der Durchführung des Bildgebungsverfahrens zu erwarten ist, wobei die Konsistenz in diesem Fall nicht vorliegt. Denkbar ist, dass während der Durchführung des Bildgebungsverfahrens Pausen bezüglich der Atmung des Patienten erforderlich sind, wobei die erforderlichen zeitlichen Längen dieser Pausen von den Parametern 18, 19 abhängen. So kann das Vorliegen der Konsistenz dann verneint werden, wenn die entsprechend resultierende zeitliche Dauer für die Atempausen eine vorgegebene, zulässige Höchstdauer überschreitet.

Ferner erfolgt das Auswerten der Eingangsdatensätze 10, 12, 14, 15, 16, 22 hinsichtlich des Erkennens des Vorliegens einer Konsistenz der geänderten Parameter 18, 19 mit den übrigen Parametern 18, 19. Konkret wird angenommen, dass die Konsistenz dann gegeben ist, wenn, insbesondere paarweise, Kombinationen von Werten für die Parameter erlaubten, vorgegebenen Kombinationen entsprechen.

Sofern im Schritt 7 die Auswertung ergibt, dass die anwenderseitige Vorgabe für die Parameter 18, 19 zu einer Inkonsistenz führt, dann erfolgt die Erzeugung einer Ausgabe 23, die auf eine weitere, zur Ausräumung dieser Inkonsistenz gerichtete Modifizierung des Protokolls 9 gerichtet ist und den Anwender konkret über die Inkonsistenz informiert. Dem Anwender wird die Ausgabe 23 über die Mensch-Maschine-Schnittstelle ausgegeben, wobei dieser sodann entscheiden kann, ob er den oder die von der Inkonsistenz betroffenen oder betroffene Parameter 18, 19 beibehalten oder ändern möchte. Konkret realisiert die Ausgabe 23 einen an den Anwender gerichteten Vorschlag bezüglich einer vorzunehmenden Änderung des jeweiligen Parameters 18, 19 zur Herstellung der fehlenden Konsistenz.

Die Ausgabe 23 ist ein für den Anwender verständlicher Text, der in einer tokenisierten Form vorliegt, bei die Parameter 18, 19, die bezüglich einer Änderung vorgeschlagen werden, im Rahmen der Token-Kombination konkret angegeben werden. Diese Vorschläge können von dem Anwender angenommen und zur automatischen Anpassung des jeweiligen Protokolls 9 weitergeleitet werden. In diesem Fall erfolgt in einem nächsten, in der Fig. 2 nicht gezeigten Schritt die finale Erzeugung des Protokolls 9 mittels der Verarbeitungseinrichtung 3.

Im Folgenden wird ein konkretes Beispiel erläutert, wie dies im Rahmen der zweiten Ausführungsform des erfindungsgemäßen Verfahrens denkbar ist. So wird zunächst der das jeweilige Standardprotokoll betreffende Eingangsdatensatz 16 abgerufen. Die gemäß diesem Standardprotokoll vorgegebenen Parameter 18, 19 werden dem Anwender über die durch die Mensch-Maschine-Schnittstelle 20 angezeigte Eingabemaske ausgegeben, der diese manuell modifizieren kann. Die hiernach vorliegenden Werte für die Parameter 18, 19 werden sodann serialisiert, also in einen zusammenhängenden Text überführt, der als der Eingangsdatensatz 22 zusammen mit den übrigen Eingangsdatensätzen 10, 12, 14, 15, 16 dem Sprachmodell 8 zugeführt wird. So kann etwa eine Echozeit, die vorliegend mit TE abgekürzt wird, anwenderseitig von 89 ms zu 50 ms geändert werden, sodass der Eingangsdatensatz 22 respektive der dem Sprachmodell 8 zurückzuführende Text wie folgt lautet: "Der Parameter TE wurde von TE=89 zu TE=50 geändert. Überprüfe, ob dieser Parameter konsistent mit der Bezeichnung des Protokolls und mit den übrigen Parametern ist und gib die Antwort ,Ja' nur dann aus, wenn dies der Fall ist. Generiere andernfalls eine Antwort in zwei Sätzen, dass und weswegen die Konsistenz nicht gegeben ist." In diesem Text werden zudem die Werte für die übrigen Parameter 18, 19 angegeben, was jedoch vorliegend aus Gründen der Übersichtlichkeit nicht konkret mit aufgeführt ist. Angenommen, dass die Bezeichnung des Protokolls eine ausschließliche T2-Wichtung impliziert, die aufgrund des geänderten Parameters TE nicht mehr vorliegt, dann kann die Ausgabe 23 wie folgt lauten: "Durch die Änderung der Echozeit von 89 ms zu 50 ms liegt bei dem neuen Protokoll im Gegensatz zu der Benennung nicht mehr ausschließlich eine T2-Wichtung vor."

Im Rahmen eines weiteren konkreten Beispiels für die zweite Ausführungsform des erfindungsgemäßen Verfahrens ist denkbar, dass die Überprüfung der Konsistenz hinsichtlich der Eingangsdatensätze 12, 14 erfolgt, die die ältere Patientenaufnahme beziehungsweise die abgerufene Patientenakte betreffen. So kann etwa anwenderseitig eine Anzahl SL vorgesehener Aufnahmeschichten von 30 auf 20 und eine Schichtdicke SLTHK von 9 mm auf 6 mm geändert worden sein, wobei der den Eingangsdatensatz 22 darstellende Text wie folgt lauten kann: "Der Parameter SL wurde auf 20 und der Parameter SLTHK auf 6 mm geändert. Überprüfe, ob für diese Parameter und für das resultierende Protokoll Konsistenz mit allen anderen Umständen im Zusammenhang mit der vorliegenden Bildgebung gegeben ist. Antworte mit ,Ja`, wenn dies der Fall ist. Generiere andernfalls eine Antwort in zwei Sätzen, dass und weswegen die Konsistenz nicht gegeben ist und mache Vorschläge, durch welche Anpassung der betroffenen Parameter Konsistenz hergestellt werden kann." Das Ergebnis der Auswertung kann die Ausgabe 23 sein, die wie folgt formuliert ist: "Es liegt eine Inkonsistenz mit früheren Patientenaufnahmen vor. Um diese Inkonsistenz auszuräumen, könnte die Schichtanzahl SL auf 30 und die Schichtdicke auf 9 mm gesetzt werden." Zusätzlich zu diesem Text können die anzupassenden Parameter 18, 19 direkt als Token-Kombinationen vorgeschlagen und anwenderseitig ausgewählt werden. Diese können mittels der Mensch-Maschine-Schnittstelle 20 zur automatischen Anpassung der jeweiligen Parameter 18, 19 weitergeleitet werden.

Nachfolgend wird das erfindungsgemäße Verfahren gemäß einem dritten Ausführungsbeispiel erläutert. Auch hierzu wird auf die in Fig. 1 gezeigte Einrichtung 1 Bezug genommen, wobei auch die anhand der Fig. 2 erläuterten Verfahrensschritte bei dem dritten Ausführungsbeispiel vorgesehen sein können. Die nachfolgend erläuterten Aspekte sind zusätzlich oder alternativ bei dem erfindungsgemäßen Verfahren gemäß dem ersten beziehungsweise zweiten Ausführungsbeispiel denkbar.

Wie bereits angesprochen wurde, stellt die medizinische Bildgebungseinrichtung 5 nur eine von mehreren Bildgebungseinrichtungen der medizinischen Einrichtung 1 dar. Grundsätzlich ist bezüglich sämtlicher Bildgebungseinrichtung in der medizinischen Einrichtung 1 vorgesehen, dass, sofern ein medizinisches Bildgebungsverfahren durchgeführt wird, ein Historienprotokoll betreffend das jeweilige Bildgebungsverfahren auf einer Historiendatenbank 24 hinterlegt wird. Folglich steht für die medizinische Einrichtung 1 mit der Historiendatenbank 24 ein Archiv zur Verfügung, das die Historienprotokolle und mithin Informationen bezüglich früherer stattgefundener Bildgebungsverfahren umfasst. Die Historiendatenbank ist mithin ebenfalls ein Teil der Bereitstellungseinrichtung 2.

Als das Historienprotokoll kann unmittelbar das zur Durchführung des Bildgebungsverfahren verwendete Protokoll hinterlegt werden. Denkbar ist auch, dass lediglich ausgewählte Informationen respektive Parameter, die bei der Durchführung des jeweiligen Bildgebungsverfahrens vorlagen, als ein das Historienprotokoll bildender Datensatz abgespeichert werden. Im Rahmen des dritten Ausführungsbeispiels ist vorgesehen, dass die im Laufe der Zeit generierten Historienprotokolle als Eingangsdatensatz 25 verwendet werden.

Diesbezüglich erfolgt die Auswertung im Schritt 7 derart, dass gegebenenfalls aufgetretene zeitliche Trends und Änderungen respektive die zeitliche Entwicklung der Parameter 18, 19 bezüglich der Durchführung der Bildgebungsverfahren erkannt und für die aktuelle Durchführung des Bildgebungsverfahrens berücksichtigt werden. So kann hierdurch vermieden werden, dass gegebenenfalls aufgetretene Änderungen bei den Parametern, die beispielsweise auf entsprechende Erfahrungen und/oder geänderte Empfehlungen zurückzuführen sind, bei der aktuell durchzuführenden Bildgebung übersehen werden.

Konkret ist auch in diesem Kontext die Auswertung im Schritt 7 auf die Überprüfung des Vorliegens von Konsistenzen gerichtet, und zwar hinsichtlich dessen, ob die vorliegenden Parameter 18, 19 mit ihren zeitlichen Entwicklungen im Einklang stehen. So ist exemplarisch denkbar, dass zu einem bestimmten Zeitpunkt ein grundsätzlich verwendeter Wert für einen der Parameter aufgrund einer Empfehlung respektive Vorgabe geändert wird, sodass diesbezüglich in einer zeitlichen Reihe, die die Werte dieses Parameters 18, 19 umfasst, ein stufenartiger Sprung auftritt. Sofern dieser Parameter 18, 19 durch den Anwender abgeändert wurde, der etwa aufgrund einer längeren Abwesenheit keine Kenntnis von dieser Änderung erlangen konnte, dann kann dies anhand des Eingangsdatensatzes 25 erkannt werden.

Im Kontext mit der Überprüfung des Vorliegens der Konsistenz ist denkbar, dass das Protokoll 9 anhand des Ergebnisses der Auswertung automatisch derart erzeugt wird, dass eine vorliegende Inkonsistenz ausgeräumt wird. Konkret ist bezüglich des dritten Ausführungsbeispiels denkbar, dass das Protokoll 9 automatisch derart erzeugt wird, dass der von der zeitlichen Änderung betroffene Parameter 18, 19 im Einklang mit dieser zeitlichen Änderung steht. Sofern als das Ergebnis der Auswertung die Ausgabe 23 erzeugt wird, dann ist denkbar, dass der Anwender analog zu dem im zweiten Ausführungsbeispiel Beschriebenen über die zeitliche Änderung informiert wird, wobei gegebenenfalls ein Vorschlag zur Ausräumung der Inkonsistenz unterbreitet wird.

Ergänzend wird angemerkt, dass die Durchführung der Konsistenzprüfung auch für die seitens der Datenbank 17 hinterlegten Standardprotokolle durchgeführt werden kann. Oft erfolgt das Einpflegen der Änderungen, die typischerweise mit einem großen Aufwand verbunden ist, nur unvollständig oder gar nicht. Die Verwendung des Sprachmodells 8 stellt eine deutliche Vereinfachung und Beschleunigung diesbezüglich dar. Insbesondere können auch etwaige Änderungen und Trends erkannt werden, die nur bezüglich bestimmter Patientendaten auftreten, also beispielsweise nur bei bestimmten Patientengruppen wie etwa Senioren oder Kindern. Das Sprachmodell 8 kann gemäß dem oben Beschriebenen über einen Prompt zur Durchführung einer spezifisch hierauf gerichteten Auswertung veranlasst werden. Der Prompt kann das Sprachmodell 8 mithin zu einer Auswertung hinsichtlich konkreter oder sämtlicher Parameter auffordern. Zu können neu zu der Datenbank 17 hinzugekommene Historienprotokolle tokenisiert werden, wobei die Tokens in einen bestehenden, vektoriellen Raum projiziert werden, wobei im Zuge der Auswertung ein Vergleich dieser Embeddings die Identifizierung etwaiger Abweichungen respektive Trends erlaubt.

Nachfolgend wird ein Ausführungsbeispiel für ein erfindungsgemäßes, computerimplementiertes Verfahren gemäß einem Ausführungsbeispiel erläutert, mittels dem das Training des Sprachmodells 8, das im Rahmen der Durchführung des Verfahrens gemäß der vorangehenden Beschreibung verwendet wird, durchgeführt wird. So werden hierzu zunächst Trainings-Eingangsdatensätze vorgegeben, die reale Eingangsdatensätze 10, 12, 14, 15, 16, 22, 25 sind, die im Zuge der Durchführung des Bildgebungsverfahrens mittels der medizinischen Einrichtung 1 bei früheren Zeitpunkten vorlagen. Ferner werden Trainings-Ergebnisse vorgegeben, die jeweils einem der Trainings-Eingangsdatensatz zugeordnet sind. Diese Trainings-Ergebnisse werden als Ideallösungen betrachtet, wobei die im Rahmen des Trainings des Modells generierten Ergebnisse hiermit verglichen werden. Zum Erhalten des trainierten Sprachmodells 8 ist die Zielvorgabe eine Minimierung der zwischen den Trainings-Ergebnissen und den generierten Ergebnissen. Das Training des Modells gemäß diesem Ausführungsbeispiel ist mithin ein überwachtes Training, wobei grundsätzlich auch die Durchführung eines nicht überwachten Trainings denkbar ist.

Überdies ist vorgesehen, dass bei dem trainierten Sprachmodell 8 auch nach dem Beginn dessen realen Einsatzes ein sogenanntes Retraining erfolgt, also eine Neuanpassung des Modells basierend auf neuen Trainingsdaten, die während des Einsatzes des Sprachmodells 8 entstehen. Hierdurch wird eine Überwachung gegebenenfalls auftretender, insbesondere zeitlicher, Entwicklungen und Trends in Echtzeit realisiert. Auch kann ein ferner vorgesehener Eingangsdatensatz erzeugt und im Rahmen des Schritts 6 vorgegeben werden, der Reaktionen respektive Rückmeldungen seitens des Anwenders betrifft, mit denen dieser auf mittels der Verarbeitungseinrichtung 3 bereitgestellten Vorschlägen reagiert hat. So kann etwa erfasst werden, wenn ein unterbreiteter Vorschlag seitens des Anwenders mehrfach abgelehnt und mithin nicht umgesetzt wurde, sodass künftig diese Änderung nicht vorgenommen beziehungsweise dieser Vorschlag nicht unterbreitet wird.

*Unabhängig vom grammatikalischen Geschlecht eines bestimmten Begriffes sind Personen mit männlicher, weiblicher oder anderer Geschlechteridentität mit umfasst.*

## Patentansprüche

1. Computerimplementiertes Verfahren zur Erzeugung eines Protokolls (9) und/oder einer Ausgabe (23), die auf eine Erzeugung oder Modifizierung eines Protokolls (9) gerichtet ist, wobei das Protokoll (9) eine Durchführungsvorschrift zur Steuerung des Ablaufs der Durchführung eines medizinischen Bildgebungsverfahrens mittels einer medizinischen Bildgebungseinrichtung (5) darstellt und wenigstens einen veränderbaren Parameter (18, 19) des Bildgebungsverfahrens zu dessen Durchführung vorgibt, wobei das Verfahren die folgenden Schritte umfasst:
- Vorgeben wenigstens eines Eingangsdatensatzes (10, 12, 14, 15, 16, 22, 25), der wenigstens eine Vorgabe für das Bildgebungsverfahren betrifft,
- Auswerten des wenigstens eines Eingangsdatensatzes (10, 12, 14, 15, 16, 22, 25), wobei in Abhängigkeit des Ergebnisses dieses Auswertens das Protokoll (9) und/oder die Ausgabe (23) erzeugt wird, wobei dieses Auswerten unter Verwendung eines trainierten und mittels eines maschinellen Lernens erzeugten Sprachmodells (8) erfolgt.

2. Verfahren nach Anspruch 1, **dadurch gekennzeichnet, dass** der Parameter (18, 19) oder wenigstens einer der Parameter (18, 19)
- ein Messparameter (18) ist, der eine während der Durchführung des Bildgebungsverfahrens vorliegende physikalische oder geometrische Gegebenheit betrifft, oder
- ein Schrittparameter (19) ist, der eine zeitliche Dauer und/oder einen Startzeitpunkt und/oder einen Endzeitpunkt wenigstens eines Messschritts betrifft, der im Rahmen der Durchführung des Bildgebungsverfahrens durchgeführt wird.

3. Verfahren nach Anspruch 1 oder 2, **dadurch gekennzeichnet, dass** der Eingangsdatensatz (10, 12, 14, 15, 16) oder wenigstens einer der Eingangsdatensätze (10, 12, 14, 15, 16)
- eine im Rahmen einer früheren Durchführung eines medizinischen Bildgebungsverfahrens erfasste Patientenaufnahme und/oder
- einen Auszug aus einer elektronischen Patientenakte und/oder
- eine auf einen aktuellen Zustand des Patienten gerichtete Patientenabfrage und/oder
- eine auf die Durchführung des Bildgebungsverfahrens gerichtete, ärztliche Vorgabe betrifft.

4. Verfahren nach einem der vorangehenden Ansprüche, **dadurch gekennzeichnet, dass** wenigstens ein hinterlegtes Standardprotokoll vorgesehen ist, bei dem der Parameter (18, 19) oder wenigstens einer der Parameter (18, 19) standardisiert vorgegeben ist, wobei der Eingangsdatensatz (16) oder wenigstens einer der Eingangsdatensätze (16) das Standardprotokoll oder wenigstens eines der Standardprotokolle ist oder umfasst.

5. Verfahren nach einem der vorangehenden Ansprüche, **dadurch gekennzeichnet, dass** der Eingangsdatensatz (22) oder wenigstens einer der Eingangsdatensätze (22) ein eine konkrete anwenderseitige Vorgabe für den Parameter (18, 19) oder für wenigstens einen der Parameter (18, 19) betrifft.

6. Verfahren nach den Ansprüchen 3 und 4, **dadurch gekennzeichnet, dass** als der Eingangsdatensatz (16, 22) oder als wenigstens einer der Eingangsdatensätze (16, 22) das Standardprotokoll oder eines der Standardprotokolle genutzt wird, das hinsichtlich des Parameters (18, 19) oder hinsichtlich wenigstens einem der Parameter (18, 19) seitens eines Anwenders geändert wurde.

7. Verfahren nach einem der vorangehenden Ansprüche, **dadurch gekennzeichnet, dass** das Auswerten des wenigstens einen Eingangsdatensatzes (10, 12, 14, 15, 16, 22, 25) auf das Erkennen des Vorliegens einer Konsistenz des wenigstens einen Parameters (18, 19) mit wenigstens einer zur Durchführung des Bildgebungsverfahrens relevanten Größe gerichtet ist.

8. Verfahren nach Anspruch 7, **dadurch gekennzeichnet, dass** das Auswerten des wenigstens eines Eingangsdatensatzes (10, 12, 14, 15, 16, 22, 25) auf das Erkennen des Vorliegens einer Konsistenz mehrerer der Parameter (18, 19) untereinander gerichtet ist.

9. Verfahren nach der Anspruche 7 oder 8, **dadurch gekennzeichnet, dass**, sofern das Auswerten des wenigstens einen Eingangsdatensatzes (10, 12, 14, 15, 16, 22, 25) ein Fehlen der Konsistenz ergibt, die Ausgabe (23) derart erzeugt wird, dass ein Anwender anhand der Ausgabe (23) über das Fehlen der Konsistenz informiert wird.

10. Verfahren nach einem der vorangehenden Ansprüche, **dadurch gekennzeichnet, dass** die medizinische Bildgebungseinrichtung (5), und gegebenenfalls wenigstens eine weitere medizinische Bildgebungseinrichtung, zu einer medizinischen Einrichtung (1) gehören, wobei im Zuge der Durchführung eines medizinischen Bildgebungsverfahrens bei der medizinischen Einrichtung (1) oder der medizinischen Bildgebungseinrichtung (5) jeweils ein Historienprotokoll generiert und gespeichert wird, wobei das Historienprotokoll das für die Durchführung dieses medizinischen Bildgebungsverfahrens benutzte Protokoll oder ein Datensatz betreffend die für die Durchführung dieses medizinischen Bildgebungsverfahrens verwendeten Parameter ist, wobei der Eingangsdatensatz (25) oder wenigstens einer der Eingangsdatensätze (25) die derart generierten Historienprotokolle ist oder umfasst.

11. Verfahren nach Anspruch 10, **dadurch gekennzeichnet, dass** das Auswerten des wenigstens eines Eingangsdatensatzes (25) auf das Erkennen einer zeitlichen Entwicklung des Parameters (18, 19) oder wenigstens einem der Parameter (18, 19) gerichtet ist.

12. Verfahren nach Anspruch 11 und nach einem der Ansprüche 7 bis 9, **dadurch gekennzeichnet, dass** das Auswerten des wenigstens eines Eingangsdatensatzes (16, 22, 25) auf das Erkennen des Vorliegens einer Konsistenz des wenigstens einen geänderten Parameters (18, 19) mit dessen zeitlicher Entwicklung gerichtet ist.

13. Medizinische Einrichtung (1) zur Durchführung des Verfahrens nach einem der vorangehenden Ansprüche, umfassend:
- eine Bereitstellungseinrichtung (2), aufweisend insbesondere eine Mensch-Maschine-Schnittstelle (20), mittels der der wenigstens eine Eingangsdatensatz (10, 12, 14, 15, 16, 22, 25) einer Verarbeitungseinrichtung (3) vorgebbar ist,
- die Verarbeitungseinrichtung (3), die dazu eingerichtet ist, das trainierte und mittels eines maschinellen Lernens erzeugte Sprachmodell (8) zum Auswerten des wenigstens eines Eingangsdatensatzes (10, 12, 14, 15, 16, 22, 25) auf diesen anzuwenden, wobei hierdurch das Protokoll (9) und/oder die Ausgabe (23) erzeugt wird.

14. Computerlesbares Speichermedium (4), umfassend Anweisungen, die, wenn diese mittels einer als ein Computer ausgebildeten Verarbeitungseinrichtung (3) ausgeführt werden, die Verarbeitungseinrichtung (3) dazu veranlassen,
- das Verfahren nach einem der Ansprüche 1 bis 12 durchzuführen oder
- wenigstens einen vorgegebenen und eine Vorgabe für ein medizinisches Bildgebungsverfahren betreffenden Eingangsdatensatz (10, 12, 14, 15, 16, 22, 25) auszuwerten, wobei in Abhängigkeit des Ergebnisses dieses Auswertens ein Protokoll (9) und/oder eine Ausgabe (23), die auf eine Erzeugung oder Modifizierung eines Protokolls (9) gerichtet ist, erzeugt wird, wobei dieses Auswerten unter Verwendung eines trainierten und mittels eines maschinellen Lernens erzeugten Sprachmodells (8) erfolgt, wobei das Protokoll (9) eine Durchführungsvorschrift zur Steuerung des Ablaufs der Durchführung eines medizinischen Bildgebungsverfahrens mittels einer medizinischen Bildgebungseinrichtung (5) darstellt und wenigstens einen veränderbaren Parameter (18, 19) des Bildgebungsverfahrens zu dessen Durchführung vorgibt.

15. Computerimplementiertes Verfahren zur Erzeugung eines trainierten Modells, das im Rahmen der Durchführung des Verfahrens nach einem der Ansprüche 1 bis 12 als das trainierte Sprachmodell (8) verwendbar ist, wobei das Verfahren die folgenden Schritte umfasst:
- Vorgeben wenigstens eines Trainings-Eingangsdatensatzes,
- Vorgeben eines Trainings-Ergebnisses, das dem wenigstens einen Trainings-Eingangsdatensatz zugeordnet wird,
- Trainieren eines Modells basierend auf dem wenigstens einen Trainings-Eingangsdatensatz und dem Trainings-Ergebnis, wodurch das trainierte Sprachmodell (8) erhalten wird.
